# EUROPÄISCHE PATENTSCHRIFT

(11) **EP 0 865 784 B1**
(45) Veröffentlichungstag und Bekanntmachung des Hinweises auf die Patenterteilung: **27.12.2017**
(21) Anmeldenummer: 98104985.1
(22) Anmeldetag: 19.03.1998
(51) Int. Cl.: A61K 6/10

(54) **Triglyceride enthaltende Abformmassen**
Impression materials containing triglyceride
Matériaux d'empreinte contenant des triglycérides

(30) Priorität: 19.03.1997 DE 19711514
(43) Veröffentlichungstag der Anmeldung: 23.09.1998
(73) Patentinhaber: 3M Deutschland GmbH, 41453 Neuss (DE)
(72) Erfinder: Bissinger, Peter, 86343 Königsbrunn (DE); Lechner, Günther, 82237 Wörtsee (DE); Wanek, Erich, 86916 Kaufering (DE)
(74) Vertreter: Brem, Roland

(56) Entgegenhaltungen:
- EP-A- 0 057 839
- EP-A- 0 566 221
- EP-A- 0 613 926
- US-A- 4 782 101

## Beschreibung

Die vorliegende Erfindung betrifft gummielastische Abform- bzw. Dubliermassen auf Basis von vulkanisierbaren Polyethermaterialien, die insbesondere im Dentalbereich, aber auch in der Orthopädie, Anwendung finden. Insbesondere beschreibt die vorliegende Erfindung vulkanisierbare Polyetherpasten mit Aziridinoendgruppen, additionsvernetzende Polyethersilikonpasten mit H-Si-Gruppen und radikalisch vulkanisierbare Polyetheracrylat- und metharylatpasten zur Herstellung genauer Abformungen bezahnter, teilbezahnter und unbezahnter Kiefer sowie von Gipsmodellen.

Aus der DE-B-17 45 810 sind Abdruckmassen von Polyethermaterialien mit Aziridinoendgruppen bekannt. Aus der DE-A1-37 41 575 und der DE-A1-38 38 587 sind Abdruckmassen auf Basis von Polyethermaterialien mit Alkenylgruppen sowie Polyorganosiloxanresten, enthaltend H-Si-Gruppen, bekannt, die unter Einwirkung von Platinkatalysatoren polymerisieren. Aus der EP-A2-0 173 085 sind Abdruckmassen von Polyethermaterialien mit Acrylat- und Methacrylatgruppen bekannt, die nach Bestrahlung mit Licht geeigneter Wellenlänge - initiiert durch den Zerfall eines Photoinitiators - polymerisieren. Ferner sind aus der DE-A1-43 06 997 Abdruckmassen auf Basis von Polyethermaterialien bekannt, die durch Zusätze hydrophiliert werden. Diese Materialien haben ein gutes Anfließvermögen an hydrophilen, oralen Flächen und damit bei diesen Anwendungen eine höhere Abdruckschärfe als andere bekannte Abdruckmassen, z.B. auf Basis von konventionellen hydroproben Silikonen.

Zur Vereinfachung der Verarbeitung dieser Abdruckmassen werden ihnen Thixotropierungsmittel, wie hochdisperse Füllstoffe, z.B. beschrieben in DE-A1-43 21 257, oder Fette verschiedenen Ursprungs, z.B. beschrieben in DE-A1-43 06 997, zugesetzt. In der DE-A1-195 05 896 ist ferner der Zusatz von hydriertem Rindertalg zu aziridinfreien Abformmaterialien beschrieben. Es ist bekannt, daß diese Thixotropierungsmittel in Polyethermaterialien mit Aziridinoendgruppen einen gewichtsmäßigen Anteil von ca. 10 % nicht überschreiten dürfen, um die mechanischen Eigenschaften der ausgehärteten Abdruckmasse nicht negativ zu beeinflussen. Dies bedeutet, daß der Anteil der aziridinohaltigen Polyether solcher Abformmassen im Bereich von 50 bis 70 Gewichtsprozent liegen muß und somit maßgeblich zur Verteuerung dieser Materialien führt. Ein weiterer Nachteil der Beimengung der bislang verwendeten Thixotropierungsmittel ist, daß die Viskosität der Polyetherpasten, die solche Thixotropierungsmittel enthalten, im Laufe der Lagerung stetig zunimmt und damit die Verwendbarkeitsdauer dieser Materialien nachteilig einschränkt. Pasten ohne diese Thixotropierungsmittel zeigen keine Tendenz zur Verstrammung.

Aufgabe der vorliegenden Erfindung ist es, Abdruckmassen auf Polyetherbasis bereitzustellen, die Thixotropierungsmittel enthalten, welche in Mengen von > 10 Gewichtsprozent zugemischt werden können, ohne daß die mechanischen Eigenschaften der ausgehärteten Massen negativ beeinflußt werden. Darüber hinaus sollen die mit diesen Thixotropierungsmitteln gefertigten Abformmassen eine möglichst geringe Zunahme der Viskosität mit zunehmender Lagerdauer zeigen.

Gelöst wird die Aufgabe durch Abformmassen, die dadurch gekennzeichnet sind, daß sie
a) ca. 30 bis 70 Gew.-% Aziridino-Polyether
b) ca. 5 bis 20 Gew.-% eines Triacylglycerides nicht tierischen Ursprungs, welches einen Stearoylgehalt ≥ 70 Gew.-%, bezogen auf den Acylgehalt des Triacylglycerids, aufweist und
c) ca. 10 bis 65 Gew.-% übliche Katalysatoren, Hilfs- und Zusatzstoffe,
jeweils bezogen auf das Gesamtgewicht der fertig angemischten Masse, enthalten.

Vorzugsweise enthalten die erfindungsgemäßen Abformmassen 30 bis 40 Gew.-% Komponente a), 5 bis 20 Gew.-% Komponente b) und 40 bis 65 Gew.-% Komponente c), jeweils bezogen auf das Gesamtgewicht der fertig angemischten Masse.

Abformmassen dieser Zusammensetzung zeichnen sich gegenüber bisher bekannten Polyetherabformmaterialien auf Aziridinbasis durch gleichbleibend gute mechanische Eigenschaften der vollständig vulkanisierten und auspolymerisierten Materialien aus (siehe Beispiele 1 und 4 sowie Tabelle 1), bei gleichzeitig deutlich niedrigerem Gehalt an Aziridino-Polyether. Dadurch lassen sich die erfindungsgemäßen Massen wesentlich kostengünstiger produzieren.

Zusätzlich lassen sich die Abformassen durch die Verwendung der Triacylglyceride nicht tierischen Ursprungs gemäß Komponente b) leichter anmischen und gestatten somit eine kürzere Produktionszeit. Überraschenderweise wurde gefunden, daß die erfindungsgemäßen Massen durch den Zusatz der beschriebenen Triacylglyceride unter Lagerung auch bei erhöhter Temperatur keine wesentliche Erhöhung der Viskosität in einem Zeitraum bis zu 24 Monaten erleiden (Beispiele 1 bis 4; sowie Tabelle 1). Dieser Effekt läßt sich jedoch nur mit solchen Triacylglyceriden nicht tierischen Ursprungs erzielen, deren Stearoylgehalt größer oder gleich 70 Gew.-%, bezogen auf den Acylgehalt des Triacylglycerids, ist. Werden in den erfindungsgemäßen Massen Triacylglyceride verwendet, deren Stearoylgehalt kleiner als 70 Gew.-%, bezogen auf den Acylgehalt des Triacylglycerids, ist, so wird bei Lagerung insbesondere bei erhöhter Temperatur manchmal ein Verstrammen beobachtet (Vergleichsbeispiel 1 bis 4; sowie Tabelle 1). Werden derartige Triacylglyceride vor ihrer Beimengung zur Abformmasse, wie in Herstellungsbeispiel 2 beschrieben, vorbehandelt (In-sich-Veresterung), so zeigen die daraus erstellten Polyetherabformmassen überraschenderweise keine wesentliche Zunahme der Viskosität bei erhöhter Temperaturlagerung

### (Beispiel 2 (Referenz) und Tabelle 1)

Die In-sich-Veresterung kann grundsätzlich durch Rühren des Fettes mit einer starken Base und anschließender Neutralisation durchgeführt werden (siehe hierzu auch "Ullmann's Encyclopedia of Industrial Chemistry", 5. Aufl., Band A10, S. 209).

Somit lassen sich zur Herstellung der erfindungsgemäßen Massen eine Vielzahl von Triacylglyceriden verwenden, wie z.B. Avocadoöl, Baumwollsaatöl, Erdnußöl, Kakaobutter, Kürbiskernöl, Leinöl, Maiskeimöl, Olivenöl, Palmöl, Reisöl, Rüböle, Saffloröl, Sesamöl, Sojaöl, Sonnenblumenöl, Traubenkernöl, Weizenkeimöl, Borneotalg, Fulwatalg, Hanföl, Illipébutter, Lupinienöle, Kandelnußöl, Kapoköl, Katiaufett, Kenafsamenöl, Kekunaöl, Mohnöl, Mowrahbutter, Okraöl, Perillaöl, Salbutter, Sheabutter und Tungöl, sofern diese Fette vor ihrer Verwendung gehärtet wurden. Als geeignet gehärtete Fette werden solche betrachtet, deren Jod-Zahl (gemessen nach der Norm DGF C-V 11b) kleiner als 20 ist. Besonders bevorzugt sind Fette, deren Jod-Zahl kleiner als 5 ist. Die Durchführung von Fetthärtungen ist beispielsweise, in "Ullmanns Enzyklopädie der industriellen Chemie", 4. Aufl., Band 11, S. 469 beschrieben. Ebenso verwendbar sind Mischungen dieser natürlich vorkommenden Fette, sowie künstlich hergestellte Fette, wie z.B. Softisan 154 oder Dynasan 118 (Fa. Hüls). Die Herstellung derartiger künstlicher Triacylglyceride ist für den Fachmann relativ einfach und kann beispielsweise aus Glycerin und den entsprechenden Fettsäuremethylestern erfolgen. Derartige Veresterungsreaktionen sind u.a. in "Houben-Weyl, Methoden der Organischen Chemie", Bd. E5/Teil 1, S. 659 ff. beschrieben.

Bevorzugte Triacylglyceride entsprechen der folgenden Formel 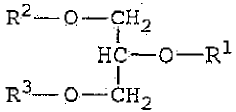 in welcher R¹, R² und R³ unabhängig voneinander C₁₁H₂₃CO, C₁₃H₂₇CO, C₁₅H₃₁CO oder C₁₇H₃₅CO bedeuten, wobei der Anteil an C₁₇H₃₅CO (Stearoyl) ≥ 70 Gew.-%, bezogen auf den Acylgehalt des Triacylglycerides, ist. Auch Gemische solcher Triacylglyceride kommen in Betracht. Beträgt der Anteil an C₁₇H₃₅CO weniger als 70 Gew.-%, so muß der Anteil dieses Restes statistisch über R¹, R² und R³ verteilt sein, damit die Lagerstabilität der Abformmasse nicht negativ beeinflußt wird. In sich veresterte Triacylglyceride natürlichen Ursprungs sowie künstlich hergestellte Triacylglyceride erfüllen die Bedingung der statistischen Verteilung von Hause aus.

Für den erfindungsgemäßen Einsatz geeignete Polyethermaterialien gemäß Komponente a) sind dem Fachmann bekannt. Besonders bevorzugt für die erfindungsgemäßen Zwecke sind die Polyethermaterialien, die in der DE-B-17 45 810 beschrieben sind.

Bevorzugte Katalysatoren gemäß Komponente c) sind in der DE-A1-25 15 593 und geeignete Verzögerer in der EP-A1-0 110 429 beschrieben. Weitere geeignete Katalysatoren sind z.B. Sulfoniumsalze, beispielsweise diejenigen, die in der US-A-41 67 618 beschrieben sind. Der angesprochene Offenbarungsgehalt der genannten Druckschriften soll hier ausdrücklich mitumfaßt sein.

Übliche Hilfs- und Zusatzstoffe gemäß Komponente c) sind Stabilisatoren, Weichmacher, Pigmente und Füllstoffe. Beispiele für solche Hilfs- und Zusatzstoffe sind u.a. in der DE-A-17 48 510 beschrieben.

Um eine vorzeitige Härtung der Abformmassen zu verhindern, müssen die Polyethermaterialien einerseits und die Katalysatoren andererseits bis zum Gebrauch voneinander getrennt gehalten werden. Vorzugsweise liegen beide Komponenten in Form von Pasten vor. Bei einer bevorzugten Ausführungsform liegen die Basiskomponenten und der Katalysator in Pastenform und räumlich voneinander getrennt vor, wobei die Basiskomponente i) 30 bis 75 Gew.-% Aziridino-Polyether, ii) 5 bis 25Gew.-% eines Triacylglycerides nicht-tierischen Ursprungs, welches einen Stearoylgehalt von ≥ 70 Gew.-% bezogen auf den Acylgehalt des Triacylglycerids aufweist,
und
iii) 0 bis 65 Gew.-% übliche Hilfs- und Zusatzstoffe enthält, und die Kathlysatorkomponente iv) 10 bis 60 Gew.-% eines üblichen Katalysators, v) 0 bis 25 Gew.-% eines Triacylglycerides gemäß Komponente ii) und vi) 15 bis 90 Gew.-% übliche Hilfs- und Zusatzstoffe enthält, wobei die Gew.-%-Angaben jeweils auf das Gesamtgewicht der Basispaste bzw. der Katalysatorpaste bezogen sind.

Die vulkanisierbaren Polyethermaterialien der einen Paste können zur Verbesserung des Anmischverhaltens mit dem im Lösungsmittel oder Weichmacher gelösten oder mittels Suspendierhilfsmittel in Suspension oder mittels Emulgatoren in Emulsion gehaltenen Polymerisationskatalysator der anderen Paste, gleichartige oder chemisch ähnliche Weichmacher oder Lösungsmittel enthalten.

Die Verwendung von Lösungen des Vernetzungsmittels in geeigneten Weichmachern, Lösungsmitteln oder Weichmacher-Gemischen ist zweckmäßig; auf diese Weise werden nicht nur extreme Mischungsverhältnisse vermieden, sondern auch feste Starter gelöst.

Übliche Weichmacher sind häufig mit den Polyethermaterialien gut verträglich. Ihre Verwendung ist nicht nur aus wirtschaftlichen Gründen, sondern auch zur Verbesserung der Eigenschaften, insbesondere zur Vermeidung oder Verringerung der Kristallisation, ratsam. Geeignet sind beispielsweise Phthalsäureester, Glykolderivate, sowie polymere Weichmacher, Sorbitanester, etc. Übliche und geeignete Weichmacher sind beispielsweise in Polyethers, Part I, herausgegeben von Norman G. Gaylord, Interscience Publishers (1963) beschrieben. Die Eigenschaften der Endprodukte sind durch Wahl eines geeigneten Ausgangsmaterials weitgehend variabel, so daß die mechanischen Werte der Endprodukte nahezu nach Maß eingestellt werden können. Der Zusatz größerer Mengen Weichmacher und/oder weiterer üblicher Zusatzstoffe kann allerdings die Wasseraufnahme, Quellung und Dimensionsänderung in einem Maß beeinflussen, daß der Abdruck unbrauchbar ist.

### Beispiele

### Herstellungsbeispiel 1 (Herstellung einer Katalysatorpaste)

32,7 g eines Sulfoniumsalzes, das gemäß Beispiel 27 der DE-A1-25 15 593 erhalten wurde, 32,2 g Acetyltributylcitrat, 5,8 g eines Block-Copolymer-Surfactants aus Propylenoxid und Ethylenoxid mit einer mittleren Molmasse von 6500, 19,1 g pyrogene Kieselsäure, 9,5 g Kieselgur und 0,7 g Pigmente werden zu insgesamt 100 g einer Paste verknetet. Diese dient in den folgenden Beispielen als Paste B.

### Vergleichsbeispiel 1

Es werden 57,9 g eines Aziridinopolyethers, der gemäß Beispiel 12 der DE-B 17 45 810 erhalten wurde, mit 11,6 g Dibenzyltoluol, 11,6 g gehärtetem Palmöl und 16,3 g Kieselgur vermischt. Nach Zugabe von 2,6 g Gemisch aus Verzögerer, Farbpigment und Aroma werden 100 g einer Paste VA1 erhalten.

Die Basispaste VAl und die Katalysatorpaste B werden im Gewichtsverhältnis 1000 g VA1 : 140 g B vollständig miteinander vermischt. Nach einigen Minuten erhält man eine gummielastische Masse.

Von der erhaltenen ausgehärteten Masse wird gemäß ISO 4823 die bleibende Deformation der Rückstellung nach Verformung mit 1,4 % sowie die Verformung unter Druck mit 2,35 % gemessen (siehe auch Tabelle 1).

Die Paste VA1 wird bei 50°C Konstanttemperatur eingelagert. Nach periodischen Zeiten wird die Viskosität mit einem Haake-Viskosimeter gemessen. Bereits nach einer Woche übersteigt die Viskosität 1300 Pa·s (Tabelle 1).

### Beispiel 1

Es werden 39,6 g eines Aziridinopolyethers, der gemäß Beispiel 12 der DE-B 17 45 810 erhalten wurde, mit 23 g Dibenzyltoluol, 17 g gehärtetem Sojaöl, 0,5 g eines Block-Copolymer-Surfactants aus Propylenoxid und Ethylenoxid mit einer mittleren Molmasse von 4400 und 17,8g Kieselgur vermischt. Nach Zugabe von 2,1 g eines Gemisches aus Verzögerer, Farbpigment und Aroma werden 100 g einer Paste A1 erhalten.

Die Basispaste A1 und die Katalysatorpaste B werden im Gewichtsverhältnis 1000 g A1 : 100 g B vollständig miteinander vermischt. Nach einigen Minuten erhält man eine gummielastische Masse.

Von der erhaltenen ausgehärteten Masse wird gemäß ISO 4823 die bleibende Deformation der Rückstellung nach Verformung mit 1,35 % sowie die Verformung unter Druck mit 2,65 % gemessen (siehe auch Tabelle 1).

Die Paste A1 wird bei 50°C Konstanttemperatur eingelagert. Nach periodischen Zeiten wird die Viskosität mit einem Haake-Viskosimeter gemessen. Noch nach zwei Jahren bleibt die Viskosität unter 1000 Pa·s (Tabelle 1).

### Vergleichsbeispiel 2

Es werden 39,6 g eines Aziridinopolyethers, der gemäß Beispiel 12 der DE-B 17 45 810 erhalten wurde, mit 21,6 g Dibenzyltoluol, 9,8 g hydriertem Rindertalg, 3,7 g eines Block-Copolymer-Surfactants aus Propylenoxid und Ethylenoxid mit einer mittleren Molmasse von 4400 und 23,6 g Kieselgur vermischt. Nach Zugabe von 1,7 g Gemisch aus Verzögerer, Farbpigment und Aroma werden 100 g einer Paste VA2 erhalten.

Die Basispaste VA2 und die Katalysatorpaste B werden im Gewichtsverhältnis 1000 g VA2 : 100 g B vollständig miteinander vermischt. Nach einigen Minuten erhält man eine gummielastische Masse.

Von der erhaltenen ausgehärteten Masse wird gemäß ISO 4823 die bleibende Deformation der Rückstellung nach Verformung mit 2,3 % sowie die Verformung unter Druck mit 3,9 % gemessen (siehe auch Tabelle 1).

Die Paste VA2 wird bei 50°C Konstanttemperatur eingelagert. Nach periodischen Zeiten wird die Viskosität mit einem Haake-Viskosimeter gemessen. Bereits nach zwei Wochen übersteigt die Viskosität 1300 Pa·s (Tabelle 1).

### Herstellungsbeispiel 2 (In-sich-Vesterung eines Fettes)

100 g gehärtetes Palmöl (z.B. PRIFAT 9835 der Fa. Unichema) werden bei 80°C aufgeschmolzen und mit 1,0 Gew.-% Natriummethylat versetzt. Man rührt bei 80°C unter Vakuum für eine Stunde, nimmt dann den Reaktionsansatz in 300 ml Toluol auf und schüttelt je einmal mit verdünnter Essigsäure und einmal mit entionisiertem Wasser aus. Nach Trocknen über Natriumsulfat entfernt man das Lösungsmittel im Vakuum und erhält 97 g in-sich-verestertes Palmöl.

### Beispiel 2 (Referenz)

Es werden 57,9 g eines Aziridinopolyethers, der gemäß Beispiel 12 der DE-B 17 45 810 erhalten wurde, mit 11,6 g Dibenzyltoluol, 11,6 g gemäß Herstellungsbeispiel 2 (siehe oben) umgeestertem Palmöl und 16,3 g Kieselgur vermischt. Nach Zugabe von 2,6 g Gemisch aus Verzögerer, Farbpigment und Aroma werden 100 g einer Paste A2 erhalten.

Die Basispaste A2 und die Katalysatorpaste B werden im Gewichtsverhältnis 1000 g A2 : 140 g B vollständig miteinander vermischt. Nach einigen Minuten erhält man eine guminielastische Masse.

Von der erhaltenen ausgehärteten Masse wird gemäß ISO 4823 die bleibende Deformation der Rückstellung nach Verformung mit 1,25 % sowie die Verformung unter Druck mit 2,6 % gemessen (siehe auch Tabelle 1).

Die Paste A2 wird bei 50°C Konstanttemperatur eingelagert. Nach periodischen Zeiten wird die Viskosität mit einem Haake-Viskosimeter gemessen. Noch nach zwei Jahren bleibt die Viskosität unter 1000 Pa·s (Tabelle 1).

### Beispiel 3

Es werden 57,9 g eines Aziridinopolyethers, der gemäß Beispiel 12 der DE-B 17 45 810 erhalten wurde, mit 11,6 g Dibenzyltoluol, 11,6 g synthetischem Triacylglycerid (SOFTISAN 154 Fa. Hüls) und 16,3 g Kieselgur vermischt. Nach Zugabe von 2,6 g Gemisch aus Verzögerer, Farbpigment und Aroma werden 100 g einer Paste A3 erhalten.

Die Basispaste A3 und die Katalysatorpaste B werden im Gewichtsverhältnis 1000 g A3 : 140 g B vollständig miteinander vermischt. Nach einigen Minuten erhält man eine gummielastische Masse.

Von der erhaltenen ausgehärteten Masse wird gemäß ISO 4823 die bleibende Deformation der Rückstellung nach Verformung mit 1,5 % sowie die Verformung unter Druck mit 2,15% gemessen (siehe auch Tabelle 1).

Die Paste A3 wird bei 50°C Konstanttemperatur eingelagert. Nach periodischen Zeiten wird die Viskosität mit einem Haake-Viskosimeter gemessen. Noch nach zwei Jahren bleibt die Viskosität unter 1000 Pa·s (Tabelle 1).

### Vergleichsbeispiel 3

Es werden 57,9 g eines Aziridinopolyethers, der gemäß Beispiel 12 der DE-B 17 45 810 erhalten wurde, mit 11,6 g Dibenzyltoluol, 11,6 g Dynasan P60 (Fa. Hüls) und 16,3 g Kieselgur vermischt. Nach Zugabe von 2,6 g Gemisch aus Verzögerer, Farbpigment und Aroma werden 100 g einer Paste VA3 erhalten.

Die Basispaste VA3 und die Katalysatorpaste B werden im Gewichtsverhältnis 1000 g VA3 : 140 g B vollständig miteinander vermischt. Nach einigen Minuten erhält man eine gummielastische Masse.

Von der erhaltenen ausgehärteten Masse wird gemäß ISO 4823 die bleibende Deformation der Rückstellung nach Verformung mit 1,8 % sowie die Verformung unter Druck mit 3,25 % gemessen (siehe auch Tabelle 1).

Die Paste VA3 wird bei 50°C Konstanttemperatur eingelagert. Nach periodischen Zeiten wird die Viskosität mit einem Haake-Viskosimeter gemessen. Bereits nach einer Woche übersteigt die Viskosität 1300 Pa·s (Tabelle 1).

### Vergleichsbeispiel 4

Es werden 57,9 g eines Aziridinopolyethers, der gemäß Beispiel 12 der DE-B 17 45 810 erhalten wurde, mit 11,6 g Dibenzyltoluol, 11,6 g hydriertem Rindertalg und 16,3 g Kieselgur vermischt. Nach Zugabe von 2,6 Gemisch aus Verzögerer, Farbpigment und Aroma werden 100 g einer Paste VA4 erhalten.

Die Basispaste VA4 und die Katalysatorpaste B werden im Gewichtsverhältnis 1000 g VA4 : 140 g B vollständig miteinander vermischt. Nach einigen Minuten erhält man eine gummielastische Masse.

Von der erhaltenen ausgehärteten Masse wird gemäß ISO 4823 die bleibende Deformation der Rückstellung nach Verformung mit 1,8 % sowie die Verformung unter Druck mit 2,6% gemessen (siehe auch Tabelle 1).

Die Paste VA4 wird bei 50°c Konstanttemperatur eingelagert. Nach periodischen Zeiten wird die Viskosität mit einem Haake-Viskosimeter gemessen. Bereits nach zwei Wochen übersteigt die Viskosität 1300 Pa·s (Tabelle 1).

### Beispiel 4

Es werden 39,6 g eines Aziridinopolyethers, der gemäß Beispiel 12 der DE-B 17 45 810 erhalten wurde, mit 21,6 g Dibenzyltoluol, 9,8 g gehärtetem Sojaöl, 3,7 g eines Block-Copolymer-Surfactants aus Propylenoxid und Ethylenoxid mit einer mittleren Molmasse von 4400 und 23,6 g Kieselgur vermischt. Nach Zugabe von 1,7 g Gemisch aus Verzögerer, Farbpigment und Aroma werden 100 g einer Paste A4 erhalten.

Die Basispaste A4 und die Katalysatorpaste B werden im Gewichtsverhältnis 1000 g A4 : 100 g B vollständig miteinander vermischt. Nach einigen Minuten erhält man eine gummielastische Masse.

Von der erhaltenen ausgehärteten Masse wird gemäß ISO 4823 die bleibende Deformation der Rückstellung nach Verformung mit 1,4 % sowie die Verformung unter Druck mit 2,7 % gemessen (siehe auch Tabelle 1).

Die Paste A4 wird bei 50°C Konstanttemperatur eingelagert. Nach periodischen Zeiten wird die Viskosität mit einem Haake-Viskosimeter gemessen. Noch nach zwei Jahren bleibt die Viskosität unter 1000 Pa·s (Tabelle 1).

**Tabelle 1: mechanische Eigenschaften und Viskositätslagerdaten der Polyetherpasten**

| Beispiel-Nr. : | Vergleichsbeispiel 1 | Beispiel 1 | Vergleichsbeispiel 2 | Beispiel 2 Referenz | Beispiel 3 | Vergleichsbeispiel 3 | Vergleichsbeispiel 4 | Beispiel 4 |
|---|---|---|---|---|---|---|---|---|
| Basispaste enthält | geh. Palmöl | geh. Sojaöl | Rindertalg | umgeest. Palmöl | Softisan | Dynasan P 60 | Rindertalg | geh. Sojaöl |
| Aziridinopolyether [Gew.-%] | 57,9 | 39,6 | 39,6 | 57,9 | 57,9 | 57,9 | 57,9 | 39,6 |
| Thixotropiermittel [Gew.-%] | 11,6 | 17 | 9,8 | 11,6 | 11,6 | 11,6 | 11,6 | 9,8 |
| Basis : Kat. | 7 : 1 | 10 : 1 | 10 : 1 | 7 : 1 | 7 : 1 | 7 : 1 | 7 : 1 | 10 : 1 |
| PE-Gehalt in Mischung [Gew.-%] | 50,79 | 36 | 36 | 50,79 | 50,79 | 50,79 | 50,79 | 36 |
| Thixotropiermittelgehalt in Mischung [Gew.-%] | 10,2 | 15,5 | 8,9 | 10,2 | 10,2 | 10,2 | 10,2 | 8,9 |
| Mech. Eigenschaften | + | + | - | + | + | - | + | + |
| Bleibende Verformung [%] | 1,4 | 1,35 | 2,3 | 1,25 | 1,5 | 1,8 | 1,8 | 1,4 |
| Elastische Verformung [%] | 2,35 | 2,65 | 3,9 | 2,6 | 2,15 | 3,25 | 2,6 | 2,7 |
| Lagerstabilität (50°C) | - | + | - | + | + | - | - | + |
| Zeit bis Viskosität > 1300 Pa^{∘}s | 1 Woche | 2 Jahre stabil | 2 Wochen | 2 Jahre stabil | 2 Jahre stabil | 1 Woche | 2 Wochen | 2 Jahre stabil |

## Patentansprüche

1. Abformmasse auf der Basis von vulkanisierbaren Polyethermaterialien, **dadurch gekennzeichnet, daß** die fertig angemischt Masse
a) ca. 30 bis 70 Gew.-% Aziridino-Polyether
b) ca. 5 bis 20 Gew.-% eines Triacylglycerides nicht-tierischen Ursprungs, welches einen stearoylgehalt ≥ 70 Gew.-%, bezogen auf den Acylgehalt des Triacylglycerids, aufweist
und
c) ca. 10 bis 65 Gew.-% übliche Katalysatoren, Hilfs- und Zusatzstoffe,
jeweils bezogen auf das Gesamtgewicht der Masse, enthält.

2. Abformmasse nach Anspruch 1, **dadurch gekennzeichnet, daß** Komponente b) aus Mischungen von Triacylglyceriden in jedem beliebigen Mischungsverhältnis besteht.

3. Abformmasse nach Anspruch 1, **dadurch gekennzeichnet, daß** Komponente b) ein künstliches Fett ist.

4. Abformmasse nach Anspruch 1, **dadurch gekennzeichnet, daß** das Triacylglycerid der folgenden Formel entspricht 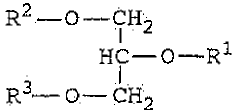 in welcher R¹, R² und R³ unabhängig voneinander die Bedeutung C₁₁H₂₃CO, C₁₃H₂₇CO, C₁₅H₃₁CO oder C₁₇H₃₅CO haben, wobei der Anteil an C₁₇H₃₅CO ≥ 70 Gew.-%, bezogen auf den Acylgehalt des Triacylglycerides, ist oder bei niedrigerem Anteil an C₁₇H₃₅CO, dieser statistisch auf die Reste R¹, R² und R³ verteilt ist.

5. Abformmasse nach Anspruch 1, **dadurch gekennzeichnet, daß** Komponente a) zu 30 bis 40 Gew.-%, die Komponente b) zu 5 bis 20 Gew.-% und die Komponente c) zu 40 bis 65 Gew.-% in der fertig angemischten Paste enthalten sind.

6. Abformmasse nach Anspruch 1, **dadurch gekennzeichnet, daß** die Basiskomponenten und der Katalysator in Pastenform räumlich getrennt voneinander vorliegen, wobei die Basiskomponente i) 30 bis 75 Gew.-% Aziridino-Polyether, ii) 5 bis 25 Gew.-% eines Triacylglycerides nicht-tierischen Ursprungs, welches einen Stearoylgehalt von ≥ 70 Gew.-% bezogen auf den Acylgehalt des Triacylglycerids aufweist
und iii) 0 bis 65 Gew.-% übliche Hilfs- und Zusatzstoffe enthält, und die Katalysatorkomponente iv) 10 bis 60 Gew.-% eines üblichen Katalysators, v) 0 bis 25 Gew.-% eines Triacylglycerides gemäß Komponente ii), und vi) 15 bis 90 Gew.-% übliche Hilfs- und Zusatzstoffe enthält, wobei die Gew.-%-Angaben jeweils auf das Gesamtgewicht der Basispaste bzw. der Katalysatorpaste bezogen sind.

7. Verwendung von Triacylglyceriden nicht-tierischen Ursprungs, welche einen Stearoylgehalt ≥ 70 Gew.-%, bezogen auf den Acylgehalt des Triacylglycerids, aufweisen, zur Herstellung einer Abformmasse auf Basis vulkanisierbarer Polyethermaterialien nach einem der Ansprüche 1-6.

## Claims

1. Molding compound based upon polyether materials that can be vulcanized, **characterized in that** the completely mixed compound contains
a) approximately 30 to 70 wt% aziridino polyether;
b) approximately 5 to 20 wt% of a triacylglyceride of non-animal origin, which has a stearoyl content ≥ 70 wt%, based upon the acyl content of the triacylglyceride; and
c) ca. 10 to 65 wt% customary catalysts, adjuvants, and additives; each based upon the total weight of the compound.

2. Molding compound according to claim 1, **characterized in that** component b) consists of mixtures of triacylglycerides in any desired mixture ratio.

3. Molding compound according to claim 1, **characterized in that** component b) is a synthetic fat.

4. Molding compound according to claim 1, **characterized in that** the triacylglyceride corresponds to the following formula 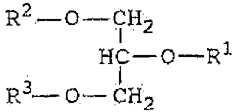 in which R¹, R², and R³, independently of one another, stand for C₁₁H₂₃CO, C₁₃H₂₇CO, C₁₅H₃₁CO, or C₁₇H₃₅CO, wherein the portion of C₁₇H₃₅CO is ≥ 70 wt%, based upon the acyl content of the triacylglyceride, or, if the portion of C₁₇H₃₅CO is lower, it is statistically distributed to the radicals, R¹, R², and R³.

5. Molding compound according to claim 1, **characterized in that**, in the completely mixed paste, component a) is contained at 30 to 40 wt%, component b) at 5 to 20 wt%, and component c) at 40 to 65 wt%.

6. Molding compound according to claim 1, **characterized in that** the base components and the catalyst are physically separate from one another in paste form, wherein the base component contains i) 30 to 75 wt% aziridino polyether, ii) 5 to 25 wt% of a triacylglyceride of non-animal origin, which has a stearoyl content of ≥ 70 wt%, based upon the acyl content of the triacylglyceride, and iii) 0 to 65 wt% of customary adjuvants and additives, and the catalyst component contains iv) 10 to 60 wt% of a customary catalyst, v) 0 to 25 wt% of a triacylglyceride according to component ii), and vi) 15 to 90 wt% customary adjuvants and additives, wherein the wt% figures are based upon the total weight of the base paste and/or the catalyst paste in each case.

7. Use of triacylglycerides of non-animal origin, which have a stearoyl content ≥ 70 wt%, based upon the acyl content of the triacylglyceride, for producing a molding compound on the basis of polyether materials that can be vulcanized according to one of claims 1-6.

## Revendications

1. Pâte d'empreinte à base de matériaux polyéther vulcanisables, **caractérisée en ce que** la pâte mélangée finie contient
a) environ 30 à 70 % en poids d'aziridine-polyéther
b) environ 5 à 20 % en poids d'un triacylglycéride d'origine non-animale, qui présente une teneur en stéaryle ≥ 70 % en poids par rapport à la teneur en acyle du triacylglycéride
et
c) environ 10 à 65 % en poids de catalyseurs, excipients et additifs courants, chacun par rapport au poids total de la pâte.

2. Pâte d'empreinte selon la revendication 1, **caractérisée en ce que** le composant b) est constitué de mélanges de triacylglycérides dans des rapports de mélange quelconques.

3. Pâte d'empreinte selon la revendication 1, **caractérisée en ce que** le composant b) est une graisse artificielle.

4. Pâte d'empreinte selon la revendication 1, **caractérisée en ce que** le triacylglycéride correspond à la formule suivante 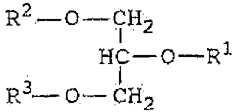 dans laquelle R¹, R² et R³ représentent indépendamment l'un de l'autre C₁₁H₂₃CO, C₁₃H₂₇CO, C₁₅H₃₁CO ou C₁₇H₃₅CO, la proportion en C₁₇H₃₅CO étant ≥ 70 % en poids par rapport à la teneur en acyle du triacylglycéride ou en cas de proportion faible en C₁₇H₃₅CO, celle-ci est répartie statistiquement entre les résidus R¹, R² et R³.

5. Pâte d'empreinte selon la revendication 1, **caractérisée en ce que** la pâte mélangée finie contient le composant a) à 30 à 40 % en poids, le composant b) à 5 à 20 % en poids et le composant c) à 40 à 65 % en poids.

6. Pâte d'empreinte selon la revendication 1, **caractérisée en ce que** les composants de base et le catalyseur sont présents sous forme pâteuse spatialement séparés les uns des autres, dans laquelle le composant de base contient i) 30 à 75 % en poids d'aziridine-polyéther, ii) 5 à 25 % en poids d'un triacylglycéride d'origine non animale, qui présente une teneur en stéaryle ≥ 70 % en poids par rapport à la teneur en acyle du triacylglycéride et iii) 0 à 65 % en poids d'excipients et d'additifs courants et le composant catalyseur iv) contient 10 à 60 % en poids d'un catalyseur classique, v) 0 à 25 % en poids d'un triacylglycéride conforme au composant ii) et vi) 15 à 90 % en poids d'excipients et d'additifs classiques, les données de % en poids étant chaque fois rapportées au poids total de la pâte de base ou de la pâte de catalyseur.

7. Utilisation de triacylglycérides d'origine non animale, qui présentent une teneur en stéaryle ≥ 70 % en poids par rapport à la teneur en acyle du triacylglycéride, pour la préparation d'une pâte d'empreinte à base de matériaux polyéther vulcanisables selon une des revendications 1 à 6.
